# EUROPEAN PATENT SPECIFICATION

(11) **EP 1 407 037 B1**
(45) Date of publication and mention of the grant of the patent: **04.01.2006**
(21) Application number: 02750839.9
(22) Date of filing: 15.07.2002
(51) Int. Cl.: C12P 19/02, C12N 9/90

(54) **PROCESS FOR MANUFACTURING OF TAGATOSE**
VERFAHREN ZUR HERSTELLUNG VON TAGATOSE
PROCEDE SERVANT A PREPARER TAGATOSE

(30) Priority: 16.07.2001 DK 200101114; 16.07.2001 US 305155 P; 16.07.2001 US 905108
(43) Date of publication of application: 14.04.2004
(73) Proprietor: Arla Foods amba, 8260 Viby J (DK)
(72) Inventor: BERTHELSEN, Hans, DK-6920 Videbaek (DK); ERIKNAUER, Kristian, DK-8260 Viby J (DK); BOTTCHER, Karen, DK-6920 Videbaek (DK); CHRISTENSEN, Hans, Jorgen, Singel, DK-6920 Videbaek (DK); STOUGAARD, Peter, DK-4050 Skibby (DK); HANSEN, Ole, Cai, DK-3500 Vaerlose (DK); JORGENSEN, Flemming, DK-2800 Lyngby (DK)
(74) Representative: Wetke, Ellen
(86) International application number: PCT/DK2002/000497
(87) International publication number: WO 2003/008617

(56) References cited:
- US-A- 5 002 612

## Description

### Technical field of the invention

The present invention concerns enzymatic manufacturing of tagatose, especially D-tagatose.

### Background of the invention

D-Tagatose is a multi-purpose low-calorie bulk sweetener having non-cariogenic and prebiotic properties . D-Tagatose can be used in food and functional food as well as in pharmaceuticals, cf. US 4786722, US 5356879 and US 5447917.

According to US 5002612 and US 5078796 D-tagatose has been manufactured by hydrolyzing lactose or a lactose containing material to a mixture of galactose and glucose using a lactase, optionally removing glucose followed by chemical isomerization of galactose to tagatose.

US 6057135 describes manufacturing of D-tagatose from cheese whey or milk, which is hydrolyzed to prepare a mixture of galactose and glucose. Glucose is separated from the galactose by fermentation of glucose and subjected to isomerization using L-arabinose isomerase.

### Summary of the invention

In a first aspect of the invention a process is provided for manufacturing of tagatose comprising a) hydrolyzing lactose or a lactose containing starting material to obtain galactose and glucose, b) isomerizing the obtained galactose with a L-arabinose isomerase, and c) chromatographic separation of products and unconverted compounds and recycling of unconverted compounds to the process.

In a second aspect of the invention a process is provided, wherein steps a) and b) are performed in one reactor.

In a further aspect of the invention a process is provided for manufacturing of D-tagatose.

In a still further aspect of the invention a process is provided, wherein the L-arabinose isomerase used in step b) is thermophilic.

In a still further aspect of the invention a process is provided, wherein the lactase used in step a) is thermophilic.

In a still further aspect of the invention a process is provided, wherein the temperature used in step(s) a) and/or b) is/are 40 - 90 °C.

Still further aspects of the invention are given in the appended claims.

### Brief description of the drawings

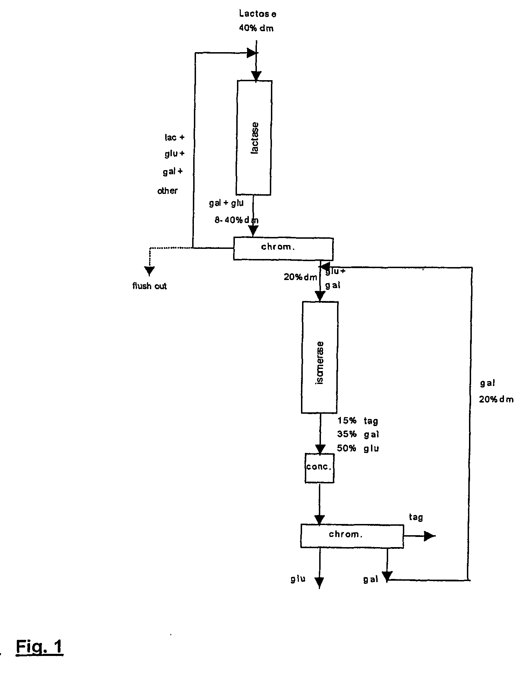

Fig. 1 shows the process of example 1 schematically,

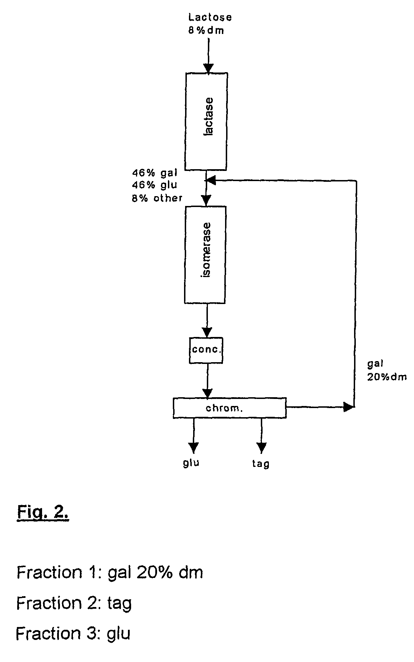

Fig. 2 shows the process of example 2 schematically, and

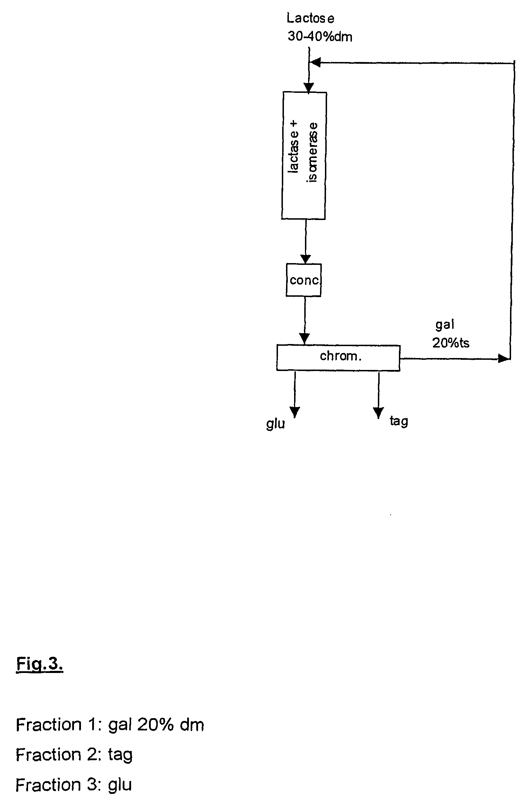

Fig. 3 shows the process of example 3 schematically.
Fig. 4 shows the result of example 4.

### Detailed description of the invention:

It has surprisingly been found that it is possible to use chromatographic separation and recycling in an enzymatic process for manufacturing of tagatose. Thereby higher yields and a cleaner process are achieved. The total yield as well as the yield for each cycle are higher.

Using this process it is possible to recycle and use any unconverted lactose and galactose. It is also possible to separate the products, tagatose and any by-products, especially glucose from galactose and use the glucose for other purposes.

The starting material can be any lactose or lactose containing material.

Lactose is a by-product from the cheese manufacture. This process gives an opportunity to convert lactose, a low-value product produced in excess, into a high value product with properties beneficial to humans. This is a way to use lactose. Opportunities for the utilization of lactose have been sought for a long period of time.

The process involves enzymatic hydrolysis of lactose, enzymatic conversion of galactose into tagatose and optionally chromatographic separation with recycling of non-converted products.

The consumption of chemicals etc is low.

The production of bi-products is low. Only one by-product is produced, viz. glucose as a glucose syrup/powder, which can be used for food.

It is possible to perform the entire reaction in one reactor containing enzymes for the hydrolysis of lactose as well as the isomerization of galactose. Thereby step a) and step b) are combined.

In so doing the complete process is improved in respect of yield per time unit. Galactose is continuously removed from the reactor by isomerization thereof to tagatose, thus reducing the concentration of galactose that would otherwise impede lactase and result in restraint of the transformation of lactose to galactose and glucose. It is possible to carry out the process at high concentration (Brix) because of the high process temperature. Evaporation capacity will be saved which again will result in improved economy as regards investment and working. The increased sugar concentration moreover has the effect that the use of preservatives can be reduced. Also the first chromatographic separation will become superfluous which again means improved economy as regards investment and running.

Especially an enzymatic conversion of lactose to glucose and galactose with subsequent isomerization of the galactose to tagatose in the same enzyme reactor has been demonstrated. The initial tests confirmed that LacS lactase enzyme and L-arabinose isomerase from T. mathranii could function under identical metal ion, buffer and temperature conditions. The principle was then tested by incubation of an 800 mM lactose solution (28%) with immobilized lactase and immobilized isomerase. Samples were analyzed for contents of lactose, glucose, galactose and tagatose by HPLC. During 24 hours of incubation the concentration of glucose increased to about 800 mM indicating that all lactose was cleaved. The concentrations of galactose and tagatose both increased linearly to about 300 mM. Consequently the degree of conversion was (300/800) x 100% = 38%.

The further enzyme can be introduced into the same reactor, a so-called combi-reactor.

The tagatose is especially D-tagatose, which is in high demand in the food industry.

All lactases can be used in step a). Examples are enzymes derived from the group consisting of *Bacillus, Sulfolobus, Thermoanaerobacter, Thermus* and *Pyrococcus.*

All L-arabinose isomerases can be used in step b). Examples are enzymes derived from the group consisting *of Bacillus, Sulfolobus, Thermoanaerobacter* and *Thermotoga.*

The enzymes can be used in any form. For example is it possible to use immobilized enzymes.

Biotechnological Institute, Denmark, can deliver usable lactases and L-arabinose-isomerases.

Biotechnological Institute, Denmark found and tested an enzyme derived from *Thermoanaerobacter mathranii, Thermoanaerobacter mathranii* DSM 11426. They have filed US patent application no. 09/905,108 covering their invention.

*K*_{*m*} values for *T. mathranii* enzyme on D-galactose are lower than values for enzymes from common L-arabinose isomerase producing organisms, such as *Aerobacter aerogenes, Bacillus amyloliquefaciens, Arthrobacter sp* , and *Lactobacillus pentosus.* Therefore *T. mathranii* has a better affinity.

This last mentioned enzyme is thermophilic.

An added benefit of using thermophilic enzymes is the possibility of using high process temperature where the solubility of lactose and glucose is higher. This means that more concentrated products can be used for the enzymatic process of the invention. This again means a less water consumption and less water for evaporation. This will give technical advantages and less need for water and energy i.a. for heating and cooling process streams.

The use of thermophilic enzymes has further made it possible to work at a higher temperature. This leads to a better hygiene because of reduced risk of contamination with damaging microorganisms. Furthermore an increased conversion of galactose to tagatose is achieved at higher temperatures compared to the conversion of arabinose to ribulose. In addition hereto there may also be technical advantages such as easier flow and quicker filtration.

It is thus preferred to use enzymes having optimal yields at high temperatures in steps a) and /or in step b). This will normally give a faster reaction. Further, it is possible to clean the system using high temperatures, for example pasteurization temperatures usually used in the dairy industry or temperatures over 100 °C, if the enzymes are thermophilic or even extremophilic. If there is no or only minor temperature difference between the temperature in steps a) and b), the energy for cooling and heating is reduced. The production process can thus be run at temperatures above 60°C. This has wide implications for the microbiology and the consumption of steam and brine for warming and cooling.

The temperature used in step(s) a) and/or b) is/are 40 - 90 °C. Normally it is 60 - 90°C. Preferably the temperature is 60 - 80 °C, more preferably 65-70 °C, and the most preferred temperature is 65 °C. However, the temperature will depend of the chosen enzymes, and it can be different in steps a) and b). As mentioned above, some advantages are achieved by using the same temperature in both steps, including performing both steps simultaneously in one reactor.

Contrary to chemical conversion of galactose into tagatose the process can be run at a pH value optimal for sugars. This significantly reduces the production of sugar degradation products. As a result, recovery and economy are improved. A typical pH value is about 7.0. Usable pH values and other reaction parameters are i.a. found in US 6057135. Yamanaka, K and Wood, W. A. (1966) *Methods in Enzymology* 9: 596-602, list a number of lactic acid bacteria providing an L-arabinose isomerase enzyme capable of producing ketoses from i.a. D-galactose.

The product of the invention in the form of syrup is so pure that it is possible to use tagatose syrups directly. Hitherto it has been necessary to purify the product, for example to crystallize the impure syrup and dissolve or solubilize it again.

As mentioned above, D-Tagatose can be manufactured from a lactose-containing source (e.g. cheese whey, casein whey or milk). Lactose is hydrolyzed to equal amounts of galactose and glucose by the lactase, which can be immobilized lactase.

Galactose is preferably separated from glucose by chromatography. Non-hydrolyzed lactose may be separated and recycled to the enzyme column. Galactose is isomerized to tagatose by optionally immobilized L-arabinose isomerase. Non-isomerized galactose may be separated by chromatography and recycled. The fraction containing D-Tagatose is crystallized. The crystals are separated from the mother liquor and dried. The mother liquor high in tagatose may be recycled/recrystallized. It is also possible to directly use the tagatose produced as syrup in food products for humans or other purposes.

Thus, there has now been found an effective enzymatic procedure combined with chromatography for manufacturing of tagatose in high yield and in a very pure form in one or two reactors. The process has special advantages if performed with thermophilic/extremophilic enzymes.

The new process for the production of tagatose is highly effective and clean due to a specific enzymatic conversion combined with recycling of non-converted products. The process is extremely effective and environmentally friendly.

### EXAMPLES

### Example 1

### Hydrolysis of lactose with recirculation Isomerization of galactose with recirculation

1. Lactose is produced from whey by ultrafiltration followed by crystallization.
2. A solution of lactose in water (8-40%DS) is hydrolyzed by immobilized lactase at high or low temperature (either by enzyme from *Aspergillus oryzae* or a thermophilic organism).
3. Glucose and galactose are separated by chromatography. Depending on concentration of feed it may be necessary to concentrate for instance by evaporation.
4. Lactose and possible galactooligosaccharides are recycled to the column containing immobilized enzyme.
5. If the concentration of oligosaccharides in the recycling loop is too high (the hydrolysis is undesirably affected), the system is flushed.
6. The fraction containing glucose and galactose is isomerized by immobilized galactose isomerase (from a thermophilic organism)
7. The mixture is concentrated for instance by evaporation
8. Tagatose is separated by chromatography
9. The tagatose fraction is concentrated and possibly crystallized
10. The glucose fraction might be concentrated for sale as a syrup or it may be further processed
11. The Galactose fraction is recycled to the isomerase column

### Example 2

### Hydrolysis of lactose without recirculation with following isomerization

1. Lactose is produced from whey by ultrafiltration followed by crystallization.
2. A solution of lactose in water (8-40%DS) is hydrolyzed by immobilized lactase (the enzyme originates from *Aspergillus oryzae*).
3. The mixture containing about 46% of glucose, 46% of galactose is passed through a column containing immobilized galactose isomerase (from a thermophilic organism). About 30% of galactose is converted into tagatose.
4. The product is separated into 3 fractions by concentration and chromatographic separation:
   - Fraction 1 contains mainly non-converted galactose. This fraction is recycled to the galactose isomerase column
   - Fraction 2 contains mainly tagatose. This fraction is concentrated for crystallization or it is marketed as syrup.
   - Fraction 3 contains mainly glucose, but also galactooligosaccharides produced by the lactase enzyme as well as un-converted lactose. This fraction is concentrated for sale as syrup or for further processing, such as crystallization or drying.

### Example 3

### Hydrolysis and isomerization in one reactor

1. Lactose is produced from whey by ultrafiltration followed by crystallization.
2. A solution of lactose in water is passed through a column containing immobilized lactase and L-arabinose isomerase (both enzymes originating from thermophilic organisms).
3. The product is separated into 3 fractions by concentration and chromatographic separation:
   - Fraction 1 contains mainly non-converted galactose. This fraction is recycled to the column for enzymatic conversion.
   - Fraction 2 contains mainly tagatose. This fraction is concentrated for crystallization or it is marketed as a syrup.
   - Fraction 3 contains mainly glucose, but also galactooligosaccharides produced by the lactase enzyme as well as un-converted lactose. This fraction is concentrated for sale as a syrup or for further processing, such as crystallization or drying

### Example 4

### One-reactor conversion of lactose to tagatose with immobilized lactase and immobilized isomerase

The β-glycosidase encoding gene from *Sulfolobus solfataricus* (Moracci M, Ciaramella M, and Rossi M. [2001] Methods in Enzymology vol. 330, p. 201-15) was cloned and expressed in *E*. *coli*. The gene was isolated by polymerase chain reaction (PCR) using purified chromosomal DNA from *Sulfolobus solfataricus* strain P2. Primers containing additional restriction sites for *Nde*l and *BamH*I were designed to yield the entire coding sequence on a fragment which was subsequently cloned into the standard expression plasmid pET3a (Novagen).

*E. coli* cells expressing the enzyme were cultivated, harvested by centrifugation, lysed in a French pressure cell and cross-linked with glutaraldehyde and polyethylenimine as described in US 4,354,105. The immobilized enzyme was recovered by centrifugation and lyophilisation of the pellet. The activity of the immobilized lactase was 1500 units/g dry weight. One unit was defined as the amount of enzyme liberating one micromole of glucose per min at 65°C, pH 7, in a 30-% (w/v) solution of lactose.

The L-arabinose isomerase gene from *Thermoanaerobacter mathranii* was cloned and expressed in *E. coli* as described in patent application no. US 09/905.108 (Biotechnological Institute, Denmark).

*E. coli* cells expressing the enzyme were cultivated, harvested by centrifugation, lysed in a French pressure cell and cross-linked with glutaraldehyde and polyethylenimine as described in US 4,354,105. The immobilized enzyme was recovered by centrifugation and lyophilisation of the pellet. The activity of the immobilized L-arabinose isomerase was 50 units/g dry weight. One unit was defined as the amount of enzyme producing one micromole of D-tagatose per min at 65°C, pH 7, in a 30-% (w/v) solution of D-galactose.

One-milliliter assay mixtures containing 20 mg of immobilized lactase, 80 mg of immobilized isomerase, 0.30 g of lactose (30%, 875 mM), 25 mM K-maleate buffer, pH 6.9, and 5 mM MnCl₂ were incubated at 65°C. A control sample without enzymes was treated similarly. Periodically, samples were taken and the concentrations of glucose, galactose and tagatose were determined by high pressure liquid chromatography using an Aminex HPX-87C column (Bio-Rad) and a refractive index detector (Waters 410). The mobile phase was de-ionized, degassed water, the column temperature was 85°C, and the flow rate was 0.6 ml/min.

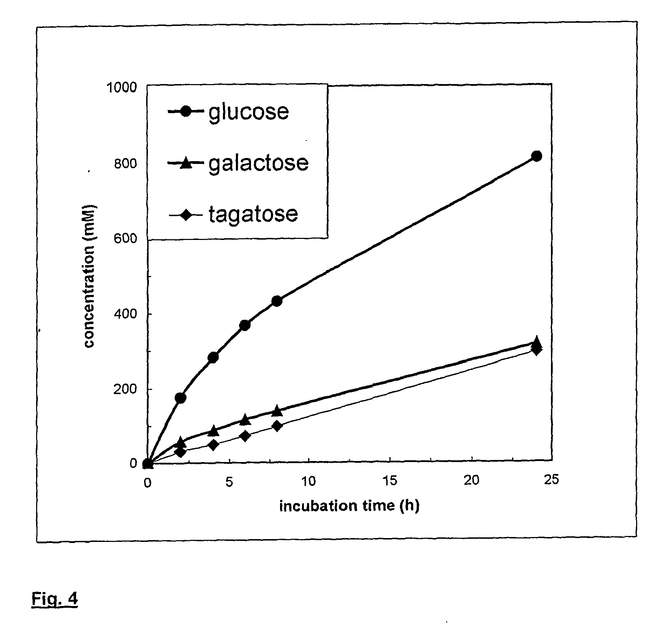

As shown in Figure 4, the concentration of glucose increased to about 800 mM over 24h, indicating that almost all lactose was hydrolyzed to galactose and glucose. The concentration of tagatose increased linearly to about 300 mM over 24h, indicating a bioconversion of 300mM/800 mM = 38%.

**TABLE Conversion of lactose to tagatose with immobilized lacS lactase from S. solfataricus and araA isomerase from T. mathranii**

| incubation time (h) | lactose (mM) | glucose (mM) | galactose (mM) | tagatose (mM) |
|---|---|---|---|---|
| 0 | 816 | 0 | 0 | 0 |
| 2 | | 176 | 57 | 30 |
| 4 | | 281 | 87 | 48 |
| 6 | | 366 | 116 | 72 |
| 8 | | 430 | 139 | 98 |
| 24 | | 811 | 316 | 295 |

## Claims

1. A process for manufacturing of tagatose comprising
a) hydrolyzing lactose or a lactose containing starting material to obtain galactose and glucose
b) isomerizing the obtained galactose with a L-arabinose isomerase, and
c) chromatographic separation of products and unconverted compounds and recycling of unconverted compounds to the process.

2. A process of claim 1, wherein unconverted lactose is separated by chromatography and recycled to step a) for hydrolysis.

3. A process of claim 1 or 2, wherein unconverted galactose is separated by chromatography and recycled to step b) for isomerization.

4. A process of any of the preceding claims, wherein step a) and step b) are performed in one reactor.

5. A process of any of the preceding claims, wherein the galactose is D-galactose and the tagatose is D-tagatose.

6. A process of any of the preceding claims, wherein the L-arabinose isomerase used in step b) is thermophilic.

7. A process of any of the preceding claims, wherein the lactase used in step a) is thermophilic.

8. A process of claim 1 or 2 wherein the L-arabinose isomerase used in step b) is derived from the group consisting of but not restricted to *Bacillus, Sulfolobus, Thermoanaerobacter* and *Thermotoga.*

9. A process of claim 8, wherein the L-arabinose isomerase is derived from *Thermoanaerobacter mathranii*.

10. A process of claim 9 wherein the L-arabinose isomerase is derived from *Thermoanaerobacter mathranii* DSM 11426.

11. A process of any of the preceding claims, wherein the lactase used in step a) is derived from the group consisting of but not restricted to *Bacillus, Sulfolobus, Thermoanaerobacter, Thermus* and *Pyrococcus.*

12. A process of claim 1, wherein one or more of the used enzymes is immobilized.

13. A process of any of the preceding claims, wherein the temperature used in step(s) a) and/or b) is/are 40 - 90 °C.

14. A process of claim 13, wherein the temperature is 60 - 90°C.

15. A process of claim 14, wherein the temperature is 60 - 80 °C.

16. A process of claim 15, wherein the temperature is 65 - 70 °C.

17. A process of claim 16, wherein the temperature is 65 °C.

18. A process according to claim 4, wherein the L-arabinose isomerase used is derived from Thermoanaerobacter mathranii and the lactase used is derived from Sulfolobus solfataricus.

## Patentansprüche

1. Verfahren zum Herstellen einer Tagatose, umfassend
a) Hydrolysieren von Lactose oder eines Lactose-enthaltenden Ausgangsmaterials, so dass Galactose und Glucose erhalten werden,
b) lsomerisieren der erhaltenen Galactose mit einer L-Arabinose-Isomerase, und
c) chromatographisches Trennen von Produkten und nicht-umgewandelten Verbindungen und Wiederzuführen von nicht-umgewandelten Verbindungen in das Verfahren.

2. Verfahren nach Anspruch 1, wobei nicht-umgewandelte Lactose durch Chromatographie abgetrennt und dem Schritt a) zum Hydrolysieren wieder zugeführt wird.

3. Verfahren nach Anspruch 1 oder 2, wobei nicht-umgewandelte Galactose durch Chromatographie abgetrennt und dem Schritt b) zum Isomerisieren wieder zugeführt wird.

4. Verfahren nach einem der vorangehenden Ansprüche, wobei Schritt a) und Schritt b) in einem einzigen Reaktor durchgeführt werden.

5. Verfahren nach einem der vorangehenden Ansprüche, wobei die Galactose D-Galactose ist und die Tagatose D-Tagatose ist.

6. Verfahren nach einem der vorangehenden Ansprüche, wobei die in Schritt b) verwendete L-Arabinose-Isomerase thermophil ist.

7. Verfahren nach einem der vorangehenden Ansprüche, wobei die in Schritt a) verwendete Lactase thermophil ist.

8. Verfahren nach Anspruch 1 oder 2, wobei die in Schritt b) verwendete L-Arabinose-Isomerase aus der Gruppe stammt, die aus *Bacillus, Sulfolobus, Thermoanaerobacter und Thermotoga* besteht, jedoch nicht darauf beschränkt ist.

9. Verfahren nach Anspruch 8, wobei die L-Arabinose-Isomerase von *Thermoanaerobacter mathranii* stammt.

10. Verfahren nach Anspruch 9, wobei die L-Arabinose-Isomerase von *Thermoanaerobacter mathranii* DSM 11426 stammt.

11. Verfahren nach einem der vorangehenden Ansprüche, wobei die in Schritt a) verwendete Lactase aus der Gruppe stammt, die aus *Bacillus, Sulfolobus, Thermoanaerobacter, Thermus und Pyrococcus* besteht, jedoch nicht darauf beschränkt ist.

12. Verfahren nach Anspruch 1, wobei eines oder mehrere der verwendeten Enzyme immobilisiert ist/sind.

13. Verfahren nach einem der vorangehenden Ansprüche, wobei die in Schritt(en) a) und/oder b) verwendete Temperatur 40 bis 90°C beträgt.

14. Verfahren nach Anspruch 13, wobei die Temperatur 60 bis 90°C beträgt.

15. Verfahren nach Anspruch 14, wobei die Temperatur 60 bis 80°C beträgt.

16. Verfahren nach Anspruch 15, wobei die Temperatur 65 bis 70°C beträgt.

17. Verfahren nach Anspruch 16, wobei die Temperatur 65°C beträgt.

18. Verfahren nach Anspruch 4, wobei die verwendete L-Arabinose-Isomerase von *Thermoanaerobacter mathranii* stammt und die verwendete Lactase von *Sulfolobus solfataricus* stammt.

## Revendications

1. Procédé de fabrication du tagatose, comprenant:
a) l'hydrolyse du lactose ou d'une matière de départ contenant du lactose pour obtenir du galactose et du glucose,
b) l'isomérisation du galactose obtenu à l'aide d'une L-arabinose-isomérase, et
c) la séparation chromatographique des produits et des composés non convertis et le recyclage des composés non convertis dans le procédé.

2. Procédé selon la revendication 1, dans lequel le lactose non converti est séparé par chromatographie et recyclé dans l'étape a) pour une hydrolyse.

3. Procédé selon la revendication 1 ou 2, dans lequel le galactose non converti est séparé par chromatographie et recyclé dans l'étape b) pour une isomérisation.

4. Procédé selon l'une quelconque des revendications précédentes, dans lequel l'étape a) et l'étape b) sont réalisées dans un réacteur.

5. Procédé selon l'une quelconque des revendications précédentes, dans lequel le galactose est le D-galactose et le tagatose est le D-tagatose.

6. Procédé selon l'une quelconque des revendications précédentes, dans lequel la L-arabinose-isomérase utilisée dans l'étape b) est thermophile.

7. Procédé selon l'une quelconque des revendications précédentes, dans lequel la lactase utilisée dans l'étape a) est thermophile.

8. Procédé selon la revendication 1 ou 2, dans lequel la L-arabinose-isomérase utilisée dans l'étape b) est dérivée du groupe constitué par *Bacillus, Sulfolobus, Thermoanaerobacter* et *Thermotoga,* mais sans s'y limiter.

9. Procédé selon la revendication 8, dans lequel la L-arabinose-isomérase est dérivée de *Thermoanaerobacter mathranii*.

10. Procédé selon la revendication 9, dans lequel la L-arabinose-isomérase est dérivée de *Thermoanaerobacter mathranii* DSM 11426.

11. Procédé selon l'une quelconque des revendications précédentes, dans lequel la lactase utilisée dans l'étape a) est dérivée du groupe constitué par *Bacillus, Sulfolobus, Thermoanaerobacter, Thermus* et *Pyrococcus,* mais sans s'y limiter.

12. Procédé selon la revendication 1, dans lequel une ou plusieurs des enzymes utilisées est(sont) immobilisée(s).

13. Procédé selon l'une quelconque des revendications précédentes, dans lequel la température utilisée dans l'étape a) et/ou l'étape b) est de 40 - 90°C.

14. Procédé selon la revendication 13, dans lequel la température est de 60 - 90°C.

15. Procédé selon la revendication 14, dans lequel la température est de 60 - 80°C.

16. Procédé selon la revendication 15, dans lequel la température est de 65 - 70°C.

17. Procédé selon la revendication 16, dans lequel la température est de 65°C.

18. procédé selon la revendication 4, dans lequel la L-arabinose-isomérase utilisée est dérivée de *Thermoanaerobacter mathranii* et la lactase utilisée est dérivée de *Sulfolobus solfataricus*.
